# EUROPÄISCHE PATENTSCHRIFT

(11) **EP 1 204 763 B1**
(45) Veröffentlichungstag und Bekanntmachung des Hinweises auf die Patenterteilung: **06.10.2004**
(21) Anmeldenummer: 00956123.4
(22) Anmeldetag: 27.07.2000
(51) Int. Cl.: C12Q 1/68

(54) **VERFAHREN ZUR CHARAKTERISIERUNG VON NUKLEINSÄUREFRAGMENTEN**
METHOD FOR CHARACTERIZING NUCLEIC ACID FRAGMENTS
PROCEDE DE CARACTERISATION DE FRAGMENTS D'ACIDE NUCLEIQUE

(30) Priorität: 28.07.1999 DE 19935749
(43) Veröffentlichungstag der Anmeldung: 15.05.2002
(73) Patentinhaber: Epigenomics AG, 10435 Berlin (DE)
(72) Erfinder: BERLIN, Kurt, D-14532 Stahnsdorf (DE)
(74) Vertreter: Schubert, Klemens, Dr.
(86) Internationale Anmeldenummer: PCT/DE2000/002595
(87) Internationale Veröffentlichungsnummer: WO 2001/009374

(56) Entgegenhaltungen:
- WO-A-00/04372
- WO-A-99/28498
- WO-A-99/29897
- DE-A- 19 625 397
- US-A- 5 510 270
- US-A- 5 929 208
- LIPSHUTZ R J ET AL: "HIGH DENSITY SYNTHETIC OLIGONUCLEOTIDE ARRAYS" NATURE GENETICS,NEW YORK, NY,US, Bd. 21, Nr. SUPPL, Januar 1999 (1999-01), Seiten 20-24, XP000865982 ISSN: 1061-4036 in der Anmeldung erwähnt
- CHEUNG V G ET AL: "MAKING AND READING MICROARRAYS" NATURE GENETICS,NEW YORK, NY,US, Bd. 21, Nr. SUPPL, Januar 1999 (1999-01), Seiten 15-19, XP000865981 ISSN: 1061-4036 in der Anmeldung erwähnt
- WARNECKE P M ET AL: "Detection and measurement of PCR bias in quantitative methylation analysis of bisulphite-treated DNA" NUCLEIC ACIDS RESEARCH,GB,OXFORD UNIVERSITY PRESS, SURREY, Bd. 25, Nr. 21, 1997, Seiten 4422-4426, XP002090424 ISSN: 0305-1048

## Beschreibung

Die Erfindung betrifft ein Verfahren zur Charakterisierung von Nukleinsäurefragmenten.

Die WO-A-99/29897 offenbart ein Verfahren und ein Kit zur Identifizierung von Nukleinsäuren. Hierbei werden massenunterscheidbare Sonden verwendet.

US-A 5,929,208 beschreibt einen Hybridisierungsassay, wobei die zu charakterisierende Nukleinsäure mit einer Oligomer Array Oberfläche kontaktiert wird, wobei komplementäre Oligomere des Arrays mit der zu charakterisierenden DNA hybridisieren und nicht hybridisierte Nukleinsäuren entfernt werden. Hierbei werden bestimmte Positionen in einem Array mit Ladungen versehen, so dass die Bindung von Oligomeren an bestimmte Orte gesteuert wird.

Es sind heute verschiedene Verfahren bekannt, mit denen man Oligonukleotid-Arrays herstellen kann. Sie lassen sich grob in 3 Gruppen einteilen:
1) Alle Oligomere werden auf herkömmliche Weise einzeln und in relativ großer Menge im Reagenzglas bzw. in den speziellen Syntheseautomaten hergestellt und danach einzeln auf den Träger aufpipettiert. Dazu verwendet man üblicherweise automatische, hochpräzise Mikropipettierroboter. Der Vorteil dieses Verfahrens ist, dass es weitgehend auf bereits optimierten Standardmethoden und - Geräten beruht. Dadurch kann man qualitativ hochstehende DNA-Arrays mit sehr reinen Oligomeren herstellen, was einen äußerst positiven Einfluß auf die mit dem Array erzielbare Detektionsempfindlichkeit und -Zuverlässigkeit hat. Der große Nachteil des Verfahrens ist, dass es enorm aufwendig und deshalb teuer ist. Dies gilt in besonderem Masse für die Synthese der einzelnen Oligomere.
   Der Kauf fertiger Oligomere stellt keine Lösung dar, da auch kommerziell synthetisierte Oligonukleotide in großen Zahlen extrem teuer sind, da die Synthese der Monomere relativ aufwendig ist, für einige spezielle Ausführungen Patentgebühren zu entrichten sind und außerdem die verwendeten Lösungsmittel aufgrund ihrer erforderlichen Reinheit teuer sind.
2) Die Oligomere werden durch pipettieren in kleinsten Mengen direkt auf dem Substrat synthetisiert. Auf jedem Rasterpunkt wird die dort vorgesehene Oligomerkette Nukleobase für Nukleobase aufgebaut. Zur Pipettierung verwendet man ähnlich wie bei Verfahren 1) einen spezialisierten Mikropipettierroboter oder z. B. eine Vorrichtung, die Kanäle zur Zuführung der einzelnen Synthesebausteine zu den jeweiligen Punkten des Arrays enthält (EP-A 0915897). Das chemische Syntheseverfahren ist grundsätzlich das gleiche wie bei der herkömmlichen Oligomer-Synthese im Syntheseautomaten. Der Unterschied ist, dass alle Oligomere unabhängig von deren Anzahl gleichzeitig, von einer einzigen automatischen Anlage direkt am vorgesehenen Bestimmungsort hergestellt werden. Die bei Methode 1) separaten Arbeitsschritte Oligomer-Synthese und Mikropipettierung sind nun zu einem einzigen Arbeitsschritt zusammengefaßt. Der Aufwand an Geräten und an manueller Arbeit wird gegenüber Methode 1) erheblich reduziert.
3) Die Oligomere werden wie bei Methode 2) direkt auf dem Substrat synthetisiert, die gezielte Anbindung der richtigen Nukleobasen an den richtigen Rasterpunkten geschieht jedoch durch eine vollkommen parallele, aus der Halbleiterfertigung stammende photolithographische Technik anstelle von sequentiellen, zielgenauen Pipettierschritten. Das Verfahren basiert darauf, dass man mit Licht einer bestimmten Wellenlänge gezielt die 5'-OH Schutzgruppen von Oligonukleotiden entfernen kann. Durch geeignete örtliche Bestrahlungsmuster kann man somit Oligonukleotid-Enden an genau jenen Rasterpunkten reaktionsfähig machen, an die man im nächsten Schritt einen neuen Nukleotidbaustein anbinden will. Bei vollständiger Benetzung der Arrayoberfläche mit einer Nukleotidbaustein-Lösung wird somit nur an den vorher belichteten Stellen eine Nukleobase angebunden, alle unbelichteten Stellen bleiben unverändert. Die örtlichen Belichtungsmuster werden erzeugt, indem man eine mikrophotographische schwarzweiß Maske zwischen dem Substrat und der Lichtquelle positioniert, die alle Rasterstellen abdeckt, die nicht reaktionsfähig gemacht werden sollen. Die Verlängerung der Oligomer-Ketten auf allen Rasterpunkten um eine Nukleobase geschieht demnach wie folgt: Mit Hilfe einer ersten Maske werden genau jene Rasterpunkt belichtet, welche um die erste der 4 möglichen Sorten von Nukleobasen (z.B. C) erweitert werden müssen. Danach wird der Array mit einer Lösung des entsprechenden Nukleotidbausteins benetzt, worauf nur die belichteten Punkte um diese Base verlängert werden. Da die neu angebundenen Nukleotidbausteine noch alle über eine Schutzgruppe verfügen, werden sie in den folgenden Schritten nicht weiter reagieren, bis ihre Schutzgruppen durch eine weitere Belichtung abgespaltet werden. Nach diesem Reaktionsschritt wird das Array gewaschen. Nun werden mit Hilfe einer zweiten Maske genau jene Rasterstellen belichtet, welche um die zweite der 4 möglichen Nukleobasen (z.B. T) erweitert werden müssen. Darauf wird das Array wiederum mit einer Lösung des entsprechenden Nukleotidbausteins benetzt und die belichteten Stellen dadurch um diese Base verlängert. Genauso verfährt man für die verbleibenden zwei Nukleobasen (G und A). Für die Verlängerung aller Oligomere um eine Nukleobase benötigt man demzufolge vier Belichtungsschritte und somit 4 Photomasken.

Diese Methode ist wegen der hohen Parallelität in der Verarbeitung sehr schnell und effizient, zudem ist sie wegen der hohen Präzision, die mit Photolithographie erreicht werden kann, gut dazu geeignet, sehr hohe Rasterdichten zu erzielen.

Eine Übersicht über den Stand der Technik in der Oligomer Array Herstellung lässt sich auch einer im Januar 1999 erschienen Sonderausgabe von Nature Genetics (Nature Genetics Supplement, Volume 21, January 1999) und der dort zitierten Literatur entnehmen.

Patente, die sich allgemein auf die Verwendung von Oligomer Arrays und photolithographisches Maskendesign beziehen, sind z. B. US-A 5,837,832, US-A 5,856,174, WO-A 98/27430 und US-A 5,856,101. Zudem existieren einige Stoff- und Verfahrenspatente, welche die Verwendung photolabiler Schutzgruppen an Nukleosiden einschränken, so z. B. WO-A98/39348 und US-A 5,763,599.

Um DNA zu immobilisieren, existieren verschiedene Verfahren. Das bekannteste Verfahren ist die Festbindung einer DNA, welche mit Biotin funktionalisiert ist, an eine Streptavidin-beschichtete Oberfläche. Die Bindungsstärke dieses Systems entspricht einer kovalenten chemischen Bindung ohne eine zu sein. Um eine Ziel-DNA kovalent an eine chemisch vorbereitete Oberfläche binden zu können, bedarf es einer entsprechenden Funktionalität der Ziel-DNA. DNA selbst besitzt keine Funktionalisierung, die geeignet ist. Es gibt verschiedene Varianten in eine Ziel-DNA eine geeignete Funktionalisierung einzuführen: Zwei leicht zu handhabende Funktionalisierungen sind primäre, aliphatische Amine und Thiole. Solche Amine werden quantitativ mit N-Hydroxy-succinimidestern umgesetzt und Thiole reagieren unter geeigneten Bedingungen quantitativ mit Alkyliodiden. Eine Schwierigkeit besteht im Einführen einer solchen Funktionalisierung in eine DNA. Die einfachste Variante ist die Einführung durch einen Primer einer PCR. Gezeigte Varianten benützen 5'-modifizierte Primer (NH₂ und SH) und einen bifunktionalen Linker.
Ein wesentlicher Bestandteil der Immobilisierung auf einer Oberfläche ist ihre Beschaffenheit. Bis jetzt beschriebene Systeme sind hauptsächlich aus Silizium oder Metall (magnetic beads). Eine weitere Methode zur Bindung einer Ziel-DNA basiert darauf, eine kurze Erkennungssequenz (z.B. 20 Basen) in der Ziel-DNA zur Hybridisierung an ein oberflächenimmobilisiertes Oligonukleotid zu verwenden.
Es sind auch enzymatische Varianten zur Einführung von chemisch aktivierten Positionen in eine Ziel-DNA beschrieben worden. Hier wird an einer Ziel-DNA enzymatisch eine 5'-NH2-Funktionalisierung durchgeführt.

5-Methylcytosin ist die häufigste kovalent modifizierte Base in der DNA eukaryotischer Zellen. Sie spielt beispielsweise eine Rolle in der Regulation der Transkription, genomischem Imprinting und in der Tumorgenese. Die Identifizierung von 5-Methylcytosin als Bestandteil genetischer Information ist daher von erheblichem Interesse. 5-Methylcytosin-Positionen können jedoch nicht durch Sequenzierung identifiziert werden, da 5-Methylcytosin das gleiche Basenpaarungsverhalten aufweist wie Cytosin. Darüberhinaus geht bei einer PCR-Amplifikation die epigenetische Information, die die 5-Methylcytosine tragen, vollständig verloren.

Es sind mehrere Verfahren bekannt, die diese Probleme lösen. Meist wird eine chemische Reaktion oder enzymatische Behandlung der genomischen DNA durchgeführt, infolge derer sich die Cytosin von den Methylcytosin Basen unterscheiden lassen. Eine gängige Methode ist die Umsetzung von genomischer DNA mit Bisulfit, die nach alkalischer Hydrolyse in zwei Schritten zu einer Umwandlung der Cytosin Basen in Uracil führt (Shapiro, R., Cohen, B., Servis, R. Nature 227, 1047 (1970)) . 5-Methylcytosin bleibt unter diesen Bedingungen unverändert. Die Umwandlung von C in U führt zu einer Veränderung der Basensequenz, aus der sich durch Sequenzierung nun die ursprünglichen 5-Methylcytosine ermitteln lassen (nur diese liefern noch eine Bande in der C-Spur).

Ein zusätzliches Verfahren zum Nachweis von 5-Methylcytosinen stellt die WO 99/28498 dar, in dem der Phänotyp von Zellmaterial bestimmt wird. Dazu wird eine genomische Gewebeprobe enzymatisch gespalten und chemisch behandelt. Fraktionen der so behandelten DNA werden mit degenerierten Oligonukleotiden amplifiziert. Dann werden die verbleibenden Cytosine auf dem Guanin-reichen DNA-Strang aus den amplifizierten Fraktionen durch PCR-Reaktion nachgewiesen. Die bei einer solchen Analyse generierten Daten können dann Rückschlüsse auf den Phänotyp des analysierten Zellmaterials gezogen werden. In diesem Verfahren werden allerdings keine Oligomer Arrays verwendet.

Eine Übersicht über die weiteren bekannten Möglichkeiten, 5-Methylcytosine nachzuweisen, ist mitsamt der dazugehörigen Literatur dem folgenden Übersichtsartikel zu entnehmen: Rein, T., DePamphilis, M. L., Zorbas, H., Nucleic Acids Res. 26, 2255 (1998).

Matrix-assistierte Laser Desorptions/Ionisations Massenspektrometrie (MALDI) ist eine neue, sehr leistungsfähige Entwicklung für die Analyse von Biomolekülen (Karas, M. and Hillenkamp, F. 1988. Laser desorption ionization of proteins with molecular masses exceeding 10.000 daltons. Anal. Chem. 60: 2299-2301). Ein Analytmolekül wird in eine im UV absorbierende Matrix eingebettet. Durch einen kurzen Laserpuls wird die Matrix ins Vakuum verdampft und das Analyt so unfragmentiert in die Gasphase befördert. Eine angelegte Spannung beschleunigt die Ionen in ein feldfreies Flugrohr. Auf Grund ihrer verschiedenen Massen werden Ionen unterschiedlich stark beschleunigt. Kleinere Ionen-erreichen den Detektor früher als größere. Die Flugzeit wird in die Masse der Ionen umgerechnet.
Technische Neuerungen der Hardware haben die Methode signifikant verbessert. Erwähnenswert ist delayed extraction (DE). Für DE wird die Beschleunigungsspannung mit einer Verzögerung zum Laserpuls eingeschaltet und dadurch eine verbesserte Auflösung der Signale erreicht, weil die Zahl der Stöße verringert wird.

MALDI eignet sich ausgezeichnet zur Analyse von Peptiden und Proteinen. Für Nukleinsäuren ist die Empfindlichkeit etwa 100-mal schlechter als für Peptide und nimmt mit zunehmender Fragmentgröße überproportional ab. Der Grund dafür liegt darin, dass für die Ionisation von Peptiden und Proteinen lediglich ein einziges Proton eingefangen werden muß. Für Nukleinsäuren, die ein vielfach negativ geladenes Rückgrat haben, ist der Ionisationsprozeß durch die Matrix wesentlich ineffizienter. Für MALDI spielt die Wahl der Matrix eine eminent wichtige Rolle. Für die Desorption von Peptiden sind einige sehr leistungsfähige Matrices gefunden worden, die eine sehr feine Kristallisation ergeben. Für DNA sind zwar mittlerweile einige ansprechende Matrices gefunden worden, jedoch wurde dadurch der Empfindlichkeitsunterschied nicht verringert. Der Empfindlichkeitsunterschied kann verringert werden, indem die DNA chemisch so modifiziert wird, dass sie einem Peptid ähnlicher wird. Phosphorothioatnukleinsäuren, bei denen die gewöhnlichen Phosphate des Rückgrats durch Thiophosphate substituiert sind, lassen sich durch einfache Alkylierungschemie in eine ladungsneutrale DNA umwandeln.

Die Kopplung eines "charge tags" an diese modifizierte DNA resultiert in der Steigerung der Empfindlichkeit in den gleichen Bereich wie er für Peptide gefunden wird. Durch diese Modifikationen hat sich auch die Möglichkeit eröffnet ähnliche Matrices, wie sie für die Desorption von Peptiden verwendet werden, zu benützen. Ein weiterer Vorteil von charge tagging ist die erhöhte Stabilität der Analyse gegen Verunreinigungen, die den Nachweis unmodifizierter Substrate stark erschweren. PNAs und Methylphosphonatoligonukleotide sind mit MALDI untersucht worden und lassen sich so analysieren.

Ein Array mit vielen tausend Ziel-DNAs kann auf einer Festphase immobilisiert und anschließend alle Ziel-DNAs gemeinsam auf das Vorhandensein einer Sequenz mittels einer Sonde (Nukleinsäure mit komplementärer Sequenz) untersucht werden.

Eine Übereinstimmung in der Ziel-DNA mit der Sonde kommt durch eine Hybridisation der zwei Teile miteinander zustande. Sonden können beliebige Nukleinsäuresequenzen von beliebiger Länge sein. Es existieren verschiedene Verfahren für die Auswahl von optimalen Bibliotheken von Sondensequenzen, welche minimal miteinander überlappen.

Sondensequenzen können auch gezielt zusammengestellt werden, um bestimmte Ziel-DNA Sequenzen aufzufinden. Oligofingerprinting ist ein Ansatz, bei welchem diese Technologie zum Einsatz kommt. Eine Bibliothek von Ziel-DNAs wird mit kurzen Nukleinsäuresonden abgetastet. Meist sind hier die Sonden nur 8-12 Basen lang. Es wird jeweils eine Sonde auf einmal an eine auf einer Nylonmembran immobilisierte Ziel-DNA-Bibliothek hybridisiert. Die Sonde ist radioaktiv markiert und die Hybridisierung wird anhand der Lokalisierung der Radioaktivität beurteilt.

Die Nutzung von Oligomer Sondenbibliotheken zur Identifizierung immobilisierter Nukleinsäuren mittels Massenspektrometrie ist beschrieben worden (EP 97 12 1471.3 und EP 97 12 1470.5). In diesem Verfahren werden allerdings keine Oligomer Arrays verwendet.

Für die Abtastung eines immobilisierten DNA-Arrays sind vielfach fluoreszent markierte Sonden verwendet worden. Besonders geeignet sind für die Fluoreszenzmarkierung ist das einfache Anbringen von Cy3 und Cy5 Farbstoffen am 5'OH der jeweiligen Sonde. Die Detektion der Fluoreszenz der hybridisierten Sonden erfolgt beispielsweise über ein Konfokalmikroskop. Die Farbstoffe Cy3 und Cy5 sind, neben vielen anderen, kommerziell erhältlich.

Aufgabe der vorliegenden Erfindung ist, ein Verfahren zur Verfügung zu stellen, welches die Nachteile des Standes der Technik überwindet.

Erfindungsgemäß wird ein Verfahren zur Charakterisierung eines Nukleinsäurefragmentes geschaffen, wobei man die folgenden Verfahrensschritte ausführt:
a) man immobilisiert das zu charakterisierende Nukleinsäurefragment auf einer Oberfläche;
b) man stellt auf einer zweiten Oberfläche einen Array von Oligomeren her, wobei die Oligomere mit einem Marker versehen sind;
c) man löst die synthetisierten Oligomere von der Oberfläche ab, ohne dass diese dabei einen vorgegebenen Bereich auf der Oberfläche zu verlassen;
d) man kontaktiert die Oberfläche, auf der die zu charakterisierende Nukleinsäure immobilisiert ist, mit der Oligomer Array Oberfläche wobei komplementäre Oligomere des Arrays an die zu charakterisierende DNA hybridisieren;
e) man entfernt nicht komplementäre Oligomere;
f) man detektiert die komplementären Oligomere mittels deren Marker, wobei man anhand des Ortes auf der Oberfläche Sequenzinformation ermittelt.

Erfindungsgemäß bevozugt ist es, dass das zu charakterisierende Nukleinsäurefragment ein Amplifikat genomischer DNA ist.

Eine besonders bevorzugte Ausführungsform des erfindungsgemäßen Verfahrens sieht vor, dass man die genomische DNA vor der Amplifikation mit einer Lösung eines Bisulfits, Disulfits oder Hydrogensulfits umsetzt.

Bevorzugt ist weiterhin, dass man das zu charakterisierende Nukleinsäurefragment kovalent an die Oberfläche bindet. Bevorzugt ist es auch, dass man in das charakterisierende Nukleinsäurefragment eine Aminofunktion einführt und diese an eine durch Silanisierung derivatisierte Glasoberfläche bindet.

Erfindungsgemäß bevorzugt ist das Verfahren ferner, wobei man die Oligomere des Arrays kovalent an die zweite Oberfläche bindet. Dabei ist es auch bevorzugt, dass man in die Oligomere des Arrays eine Aminofunktion einführt und diese an eine durch Silanisierung derivatisierte Glasoberfläche bindet.

Es ist außerdem bevorzugt, dass man den Oligomer Array durch Festphasensynthese der Oligomere auf der zweiten Oberfläche herstellt.

Besonders bevorzugt ist es, dass man die Festphasensynthese der Oligonukleotide auf der zweiten Oberfläche in einer geschlossenen Synthesekammer durchführt, in welche man selektiv Synthesereagenzien einspeist. Hierbei ist insbesondere bevorzugt, dass man die Synthese der Oligomere durch selektive Zuführung der Synthesereagenzien an die jeweiligen Orte, an denen die Oligomere synthetisiert werden, durchführt.

Erfindungsgemäß bevorzugt ist es ferner, dass man photolithographische Methoden und photolabile Schutzgruppen zur Oligomersynthese verwendet.

Weiterhin ist auch bevorzugt, dass man für das photolithographische Verfahren elektronisch ansteuerbare und/oder veränderbare Masken verwendet. Hierbei ist besonders bevorzugt, dass man für die photolithographische Verfahren ein selektiv schaltbaren Spiegelarray zur Erzeugung eines Belichtungsmusters verwendet.

Weiterhin ist es erfindungsgemäß bevorzugt, dass man die Synthese der Oligomere in einem Array von Kavitäten durchführt, welche man gegebenenfalls weiterhin als Kammern für die Hybridisierung verwendet.

Eine bevorzugte Variante des erfindungsgemäßen Verfahrens ist ferner, dass man die Immobilisierung der zu charakterisierenden Nukleinsäurefragmente an einen Array von Kavitäten durchführt, welche man gegebenenfalls weiterhin als Kammern für die Hybridisierung verwendet.

Dabei ist es bevorzugt, dass man als Marker der Oligomere chemische Gruppen verwendet, welche eine Massenveränderung und/oder Fluoreszenz bewirken. Insbesondere bevorzugt ist es bei dem erfindungsgemäßen Verfahren, dass man die Detektion der hybridisierten Oligomere mittels Massenspektrometrie, vorzugsweise mittels Matrix assistierter Laser Desorptions/Ionisations Massenspektrometrie (MALDI), durchführt.

Für die Durchführung des Verfahrens kann ein Kit verwendet werden, umfassend Reagenzien und/oder Referenz-Nukelinsäurefragmente und/oder Referenz-DNA und/oder behandelte Oberflächen und/oder photolithographische Masken und/oder Oligomere.

Die vorliegende Erfindung also beschreibt ein Verfahren zur Charakterisierung von Nukleinsäuren. In einer besonders bevorzugten Variante dient es zur Identifizierung von Cytosin Methylierungsmustern in Amplifikaten genomischer DNA.

Die Erfindung betrifft somit ein Verfahren zur Charakterisierung von Nukleinsäuren. Dazu wird die Nukleinsäure auf einer Oberfläche immobilisiert. Auf einer zweiten Oberfläche wird ein Array von markierten Oligomeren, bevorzugt Oligonukleotiden, mittels Festphasensynthese hergestellt und anschließend die Oligomere von der Oberfläche abgespalten, ohne dass diese einen vorgegebenen Bereich auf der zweiten Oberfläche verlassen können. Die beiden Oberflächen werden nach der Zugabe einer geringen Menge einer Pufferlösung kontaktiert und die synthetisierten Oligomere hybridisieren an den Orten, an denen auch ihre Synthese stattfand, an die auf der ersten Oberfläche immobilisierte Nukleinsäure. An den Orten, an denen eine Hybridiserung stattfand, können nachfolgend die Oligomere über ihre Marker registriert werden. Das Muster von hybridisierten Oligomeren, das dabei erhalten wird, wird für die Ermittlung von Sequenzinformation in der zu charakterisierenden Nukleinsäure verwendet. In einer besonders bevorzugten Variante dient das Verfahren zur Identifizierung von Cytosin-Methylierungsmustern in genomischer DNA. Dazu wird diese mit einer Bisulfitlösung behandelt und amplifiziert, bevor sie auf die erste Oberfläche aufgebracht wird.

Zur Charakterisierung einer amplifizierten genomischen DNA Probe werden Oligomer Arrays eingesetzt, indem die DNA Probe fluoreszenzmarkiert wird und an einen solchen Array von immobilisierten Oligomeren hybridisiert wird (Stand der Technik). Ebenso ist es möglich, einen Array von Amplifikaten herzustellen und daran Sonden zu hybridisieren. Jedoch können dafür nur wenige Sonden gleichzeitig verwendet werden, da sie sich anhand z. B. ihrer Fluoreszenz nur eingeschränkt unterschieden lassen. Ein Patent, dass dieses Problem mittels Massenspektrometrie umgeht, ist im Stand der Technik erwähnt.

Diese Erfindung beschreibt die umgekehrte Variante, die zu identifizierende DNA auf der Oberfläche zu immobilisieren, und dennoch die Vorteile der Oligomer Array Technologie zu nutzen. Sie bietet zahlreiche Vorteile. Zum einen kann die Hybridisierung wesentlich schneller durchgeführt werden, da bei der hier beschriebenen Variante die kurzen Oligonukleotide über eine extrem geringe Distanz zu der immobilisierten Ziel DNA diffundieren können und diese zudem in einem großen Überschuß vorliegen können. Zum anderen kann der Chip mit der kostbaren immobilisierten Ziel DNA so häufig wie für ihre vollständige Charakterisierung notwendig nach dem Entfernen der Oligomere wiederverwendet werden. Es ist auch in vielen Fällen einfacher, Marker an die Oligomere anzubringen als an die oft in wesentlich geringeren Mengen vorliegende amplifizierte DNA. Dies gilt insbesondere, wenn die Detektion mittels Massenspektrometrie erfolgt, da in diesem Fall die Oligomere direkt detektiert werden können. Diese Variante erlaubt auch die Verwendung von Oligomer Bibliotheken, deren Bestandteile alle über ihre Masse unterscheidbar sind.

Ein weiterer, wesentlicher Vorteil der vorliegenden Erfindung ist, dass die Oligomere nicht mehr unbedingt in unmittelbarer Nähe der Oberfläche an die Ziel DNA hybridisieren, wie es sonst bei Arrays immobilisierter Oligomere zwangsläufig der Fall ist. Dies verursacht häufig Probleme vor allem hinsichtlich der Hybridisierungseffizienz. Zugleich bleiben jedoch bei der vorliegenden Erfindung die allgemeinen Vorteile der Oligomer-Array Synthese, wie als Stand der Technik beschrieben, erhalten.

Das erfindungsgemäße Verfahren zur Charakterisierung von Nukleinsäuren setzt sich aus den folgenden Schritten zusammen:
1. Eine zu charakterisierende Nukleinsäure wird auf einer Oberfläche immobilisiert.
2. Auf einer zweiten Oberfläche wird ein Array von Oligomeren hergestellt, die mit einem detektierbaren Marker versehen sind.
3. Die synthetisierten Oligomere werden von der Oberfläche abgelöst, ohne dabei einen vorgegebenen Bereich auf der Oberfläche zu verlassen.
4. Die Oberfläche, auf der die zu charakterisierende Nukleinsäure immobilisiert wurde, wird mit der Oligomer Array Oberfläche kontaktiert.
5. Komplementäre Oligomere des Arrays hybridisieren an die zu charakterisierende DNA, und nicht komplementäre Oligomere werden durch Waschschritte entfernt.
6. Die komplementären Oligomere werden über ihre Marker detektiert und anhand ihres Ortes auf der Oberfläche Sequenzinformation ermittelt.

Die zu charakterisierende Nukleinsäure kann genomische DNA, bevorzugt amplifizierte Fragmente einer genomischen DNA Probe oder auch RNA sein. In einer besonders bevorzugten Variante des Verfahrens erfolgt zuvor eine Behandlung der genomischen DNA-Probe mit einer Bisulfitlösung, um eine Umwandlung von Cytosin in Uracil zu bewirken, während Methylcytosin unter diesen Bedingungen nicht umgesetzt wird. Mit diesem vorangehenden Schritt ist es möglich, das Verfahren für die Identifizierung von Methylierungsmustern in genomischer DNA zu verwenden. Die genomische DNA Probe wird bevorzugt, um die Sensitivität des Verfahrens zu verbessern, amplifiziert. Dies kann besonders bevorzugt mittels PCR erfolgen.

Im ersten Schritt des Verfahrens wird nun die zu charakterisierende DNA auf einer Oberfläche immobilisiert. Diese Oberfläche kann aus Glas, Quarzglas, oder auch beispielsweise Silicium bestehen. In einer bevorzugten Variante des Verfahrens wird die Oberfläche chemisch aktiviert, so dass eine oder mehrere kovalente Bindungen der zu charakterisierenden Nukleinsäure zu der funktionalisierten Oberfläche geschaffen werden können. Diese Aktivierung der Oberfläche wird bevorzugt durch Silanisierung durchgeführt. Dabei kommt zum einen eine Aktivierung mit Epoxyfunktionen in Betracht, an die die zu charakterisierende DNA entweder direkt oder aber bevorzugt über eine in der PCR über einen Primer eingefügte primäre Aminofunktion bindet. Zum anderen kann die Oberfläche mittels Silanisierung aminofunktionalisiert werden. Die zu charakterisierende DNA kann dann über ein Linkermolekül mit dieser Aminofunktion verknüpft werden. Die zu charakterisierende DNA trägt in diesem Fall bevorzugt in der PCR angefügte Amino- oder Mercaptofunktionen. Als bifunktionale Linkermoleküle werden z. B. SIAB, DMS oder PITC verwendet.

Hinsichtlich der Vorbehandlung der Oberfläche und der Linkermethodik gilt gleiches für den zweiten Schritt des Verfahrens, der Herstellung der Oligomer Arrays auf der zweiten Oberfläche. Die Oligomer-Arrays werden effizient, wie es Stand der Technik ist, hergestellt, jedoch mit dem Unterschied, dass sie von der Festphase wieder abspaltbar sind. Diese Abspaltung kann entweder photochemisch oder durch die Einwirkung einer Säure oder Base, bevorzugt einer Base, erfolgen. Die Oberfläche kann für die Herstellung der Oligomer Arrays sowohl mit primären Alkohol oder Aminofunktionen derivatisiert werden. Die primären Alkoholfunktionen werden dem Stand der Technik entsprechend mittels Epoxidierung der Oberfläche und nachfolgende Umsetzung mit einem Oligoethylenglykol, bevorzugt wird jedoch die kürzlich von Beier et al. (Nucleic Acids Research, 1999, S. 1970-77) vorgestellte Aminofunktionalisierung von Glasoberflächen durchgeführt. Um eine photochemische Abspaltung durchführen zu können, muß nach der Amino- oder OH-funktionalisierung bevorzugt zunächst ein photospaltbarer Linker eingefügt werden. In einer weiteren Ausführung des Verfahrens kommt für die Abspaltung der Oligomere nach der Synthese die von Beier und Pfleiderer vergestellte Schutzgruppenstrategie zum Einsatz, wobei für die Abspaltung der Oligomere von der Oberfläche die Base DBU verwendet wird (DE-A 196 25 397).

Die Synthese der Oligomerarrays erfolgt in einer besonders bevorzugten Variante des Verfahrens in einer geschlossenen Synthesekammer, in welche die Synthesereagenzien selektiv eingespeist werden können. Die Monomere werden jeweils direkt an die jeweiligen Syntheseorte geleitet, bevorzugt durch ein Pin-Tool oder über einen Piezo-Pipettierroboter, wobei die Synthesekammer für diesen Schitt geöffnet wird. Alternativ kann die Synthese mittels photolithographischer Methoden erfolgen, wie auch im Stand der Technik beschrieben. Dabei wird über eine Maske nur den Bereichen Licht zugeleitet, in den einer Kettenverlängerung des Oligomers um jeweils eine bestimmtes Monomer erfolgen soll (siehe Stand der Technik). Besonders bevorzugt ist dabei die Verwendung dynamischer Masken, die durch elektronische Ansteuerung veränderbar sind. Diese Masken können entweder ein LCD Display, Mikroverschlußarrays oder ein schaltbares Glasfaserbündel sein. Zudem können über Arrays von kippbaren Mikrospiegeln, wie sie in modernen Projektoren verwendet werden, Punkten auf der Oberfläche selektiv Licht zugeführt werden. Alternativ können viskoelastische Mikrospiegel eingesetzt werden. Die photolithographischen Verfahren erfordern die Verwendung photolabiler Schutzgruppen an den Monomeren, während bei der Zuführung der Monomere mittels z. B. eines Pipettiersystems herkömmliche Schutzgruppen verwendet werden können.

Es ist auch möglich, an einem oder mehreren Orten auf der zweiten Oberfläche mehrere unterschiedliche Oligomere oder Oligomer-Bibliotheken zu synthetisieren, sofern sie nach der Hybridisierung alle mittels der verwendeten Detektionsmethode unterscheidbar sind.

Die Oligomer Arrays können aus Oligonukleotiden und/oder PNAs (Peptide Nucleic Acids) zusammengesetzt sein. Hinsichtlich der prinzipellen Vorgehensweise bei der Synthese ändert dies wenig, es werden jeweils die dem Stand der Technik entsprechenden Syntheseverfahren angewandt. Werden jedoch Bibliotheken an einem oder mehreren Punkten des Arrays verwendet, so wird bevorzugt PNA eingesetzt, da hier leichter eine Massenunterscheidbarkeit der resultierenden Bestandteile der Bibliothek erzielt werden kann. Die Oligomere werden bei der Arraysynthese mit einer Markierung versehen, die bevorzugt entweder ein Fluoreszenzfarbstoff oder aber eine bestimmte Masse sein kann. Die Masse kann entweder die des Oligomers selbst oder aber die des Oligomers zuzüglich einer Massenmarkierung in Form einer funktionellen Gruppe sein.

Im dritten Punkt des Verfahrens wird nun eine Abspaltung der Oligomere von der Oberfläche durchgeführt. Dabei ist zu beachten, dass die Abspaltung nicht zu einer lateralen Bewegung dieser Oligomere auf der Oberfläche führen darf, wobei die Ordnung des Arrays zerstört würde. Um dies zu verhindern, wird in einer besonders bevorzugten Variante des Verfahrens bereits die Synthese des Oligomer Arrays so durchgeführt, dass die Synthese eines jeden Typs von Oligomer in einer ihm zugeordneten Kavität stattfindet. Das Oligomer verläßt diese Kavität auch nach dessen z. B. photochemischer Abspaltung von der Oberfläche nicht.

Schließlich werden im vierten Punkt des Verfahrens die abgelösten, aber immer noch in der ursprünglichen zweidimensionalen Anordnung vorliegenden markierten Oligomere an die zu charakterisierende zu charakterisierende Nukleinsäure hybridisiert. Dies erfolgt in einer besonders bevorzugten Variante des Verfahrens dadurch, dass in die Kavitäten der zweiten Oberfläche, in denen sich die Oligomere befinden, Hybridisierungspuffer gegeben wird und anschließend die erste Oberflache mit der zu charakterisierenden Nukleinsäure mit der zweiten kontaktiert wird. Alternativ kann auch ausschließlich die erste Oberfläche oder beide Oberflächen Kavitäten enthalten, die dann jeweils mit Hybridisierungspuffer gefüllt werden. Dies kann beispielsweise bevorzugt mit einem Pipettierroboter oder einem Pin-Tool Roboter erfolgen. Das Raster der Kavitäten entspricht bevorzugt dem Raster von Oligomeren im Array auf der zweiten Oberfläche.

Nach der Hybridisierung der komplementären Oligomere an die zu charakterisierende Nukleinsäure werden die beiden Oberflächen voneinander getrennt und die nicht komplemnetären Oligomere von der ersten Oberfläche durch Waschschritte entfernt.

Im sechsten Punkt des Verfahrens werden die komplementären Oligomere über ihre Markierungen detektiert. Aus der Arraysynthese ist bekannt, an welchem Punkt auf der ersten Oberfläche welche Oligomere hybridisiert haben können. Die Markierungen sind in einer besonders bevorzugten Variante des Verfahrens Fluoreszenzfarbstoffe, besonders bevorzugt wiederum Cy3 oder Cy5. In einem Fluoreszenz-Scanner kann nun die Fluoreszenz an jedem Ort auf der Oberfläche detektiert werden und mit der vorhandenen Ortsinformation die Identität der hybridisierten Oligomere bestimmt werden. Daraus wiederum wird vollständige oder partielle Sequenzinformation über die zu charakterisierende DNA erhalten.

In einer besonders bevorzugten Variante des Verfahrens ergibt die Identität der hybridisierten Oligomere Information über Cytosin Methylierungen in der zu charakterisierenden DNA, falls diese vor Schritt 1 dieses Verfahrens mit einer Bisulfitlösung behandelt wurde.

In einer weiteren bevorzugten Variante des Verfahrens wird die Detektion der hybridisierten Oligomere mittels Massenspektrometrie, bevorzugt Matrix-assistierter Laser Desorptions/Ionisations Massenspektrometrie (MALDI-MS), durchgeführt. Dazu wird zunächst die erste Oberfläche mit den hybridisierten Oligomeren mit einer Matrix beschichtet und anschließend die jeweiligen Punkte im Massenspektrometer direkt angefahren. Die Software des Massenspektrometers erlaubt dabei die Zuordnung der erhaltenen Massenspektren zu den jeweiligen Punkten auf der Oberfläche. Die hybridisierten Oligomere werden über ihre Massen eindeutig identifiziert. In einer bevorzugten Variante werden die Oligomere in der Arraysynthese mit Massenlabeln versehen. Dies sind Funktionalitäten, die nur eine Erhöhung der Masse des Oligomers um einen bestimmten Betrag zum Ziel haben, um dessen Unterscheidbarkeit von Oligomeren anderer Sequenz zu sichern. So können auch an einem Punkt auf der zweiten Oberfläche von vorneherein mehrere unterschiedliche Oligomere oder auch Oligomerbibliotheken synthetisiert worden sein, wenn ihre Massenunterscheidbarkeit an jeweils mindestens einem Punkt des Arrays gesichert ist.In einer besonders bevorzugten Variante des Verfahrens ergibt die Identität der hybridisierten Oligomere wiederum Information über Cytosin Methylierungen in der zu charakterisierenden DNA, falls diese vor Schritt 1 dieses Verfahrens mit einer Bisulfitlösung behandelt wurde. In einer besonders bevorzugten Variante des Verfahrens wird diese Information in eine Datenbank eingegeben und mit dem zur charakterisierenden DNA gehörigen Phänotyp korreliert.
Die folgenden Beispiele erläutern die Erfindung:

### Beispiel 1:

### Synthese von DNA mit 5'-DMT und photolabilen Schutzgruppen

Die Synthese von DNA Oligomeren erfolgt nach dem etablierten Phosphoramidit-verfahren, hier auf modifizierten Glasoberflächen. Als 5'-Schutzgruppen können die säurelabile DMT-Gruppe oder photoabspaltbare Gruppen verwendet werden, welche an sich bekannt sind. Zur Abspaltung dieser Gruppen werden im Falle des DMT's eine Lösung von Trichloressigsäure in Dichlormethan, im Falle der photolabilen Gruppen Licht mit geeigneter Wellenlänge, bevorzugt zwischen 320 und 365 nm, verwendet. Die Abspaltung der Schutzgruppen auf den exocyclischen Aminen und den Phosphaten erfolgt nach Standardverfahren mit konzentriertem Ammoniak.

### Beispiel 2:

### Synthese von PNA Oligomerarrays

Die Synthese von PNA Oligomeren (als Oligomerarray) wird durch Spotten der einzelnen Komponenten auf den Chip durchgeführt. Nach dem Spotten eines Monomers oder eines Reagenzes wird der Chip mit Dimethylformamid gewaschen, um nicht umgesetzte Chemikalien zu ertfernen. Dabei wird z. B. die PNA Sequenz 5'-AGC CAG CTC ACT ACC TAG-3' vom C-Terminus (3') hin zum N-Terminus (5') aufgebaut. Die Synthese erfolgt durch Binden der Säurefunktion des N-geschützten Monomers G (Fmoc, 5 Äquivalente) in einer DMF-Lösung zusammen mit 5 Äquivalenten DIPEA, 7.5 Äquivalenten Lutidin und 4.5 Äquivalenten HATU auf die modifizierte Glasoberfläche. Die 5'-Amino-Gruppen der Monomere A, C, G und T sind mit einer Fmoc-Gruppe, die exocyclischen Aminogruppen auf den Monomeren A, C und G sind mit einer Bhoc-Gruppe geschützt. PNA Monomere und Chemikalien sind kommerziell (z. B. Perkin Elmer) erhältlich.

Anschließend wird mit DMF gewaschen, nicht umgesetzte NH₂-Gruppen mit einem Gemisch aus Acetanhydrid/Lutidin in DMF gecappt, mit DMF gewaschen und die geschützte 5'-Aminofunktion mit 20 % Piperidin in DMF entschützt. Nach dem Waschen mit DMF wird das A-Monomer auf das gebundene G-Monomer gespottet. Die vollständige PNA-Sequenz wird durch die nachfolgenden Synthesezyklen synthetisiert. Die Bhoc-Gruppen der exocyclischen Aminogruppen der Basen A, C und G werden am Ende der Synthese mit Trifluoressigsäure unter Anwesenheit von m-Kresol entfernt.

### Beispiel 3:

### Synthese von DNA auf Chips, die einen photoabspaltbaren Linker tragen

Im ersten Schritt werden die Glassubstrate chemisch modifiziert, so dass eine gezielte Anbindung von Oligonukleotidbausteinen erfolgen kann, wie es Stand der Technik ist. Durch Aufbringen von verschiedenartigen Oligonukleotiden auf ein Substrat lassen sich üblicherweise Oligonukleotid-Arrays herstellen. Dazu werden die Substrate silanisiert, wobei das Silan eine funktionalisierte (beispielsweise OH- oder NH₂-) Alkylkette trägt. Anschließend wird die Oberfläche mit einem photolabilen Linker versehen, beipielsweise 4[4-1-(Fmoc-aminomethyl)-2-methoxy-5-nitrophenoxybutansäure (Novabiochem). Dieser Linker erlaubt eine Abspaltung der Oligonukleotide unter Einstrahlung von Licht der Wellenlänge 365 nm. Die Synthese der Oligonukleotide, beispielsweise der Sequenz 5'-AGC CAG CTC ACT ACC TAG-3', erfolgt wie im Beispiel 1 für DMT beschrieben wurde auf der entschützen Aminofunktion des Linkers. Schutzgruppen der exocyclischen Amine, beipielsweise Benzoyl oder Isobutyryl, werden mit konzentriertem Ammoniak entfernt. Die Abspaltung der DNA vom gesamten Chip wird photochemisch durchgeführt und anschließend von der Oberfläche gewaschen.

### Beispiel 4:

### Synthese von PNA auf Chips, die einen photoabspaltbaren Linker tragen

Die Modifizierung der Oberfläche des Glassubstrates erfolgt wie in Beispiel 3 beschrieben mit einem gespotteten photolabilen Linker. Die Synthese der PNA Sequenz, beispielsweise 5'-AGC CAG CTC ACT ACC TAG-3', wird durch Spotten der Komponenten wie in Beispiel 2 beschrieben auf der entschützten Aminofunktion des photolabilen Linkers durchgeführt. Die Bhoc Schutzgruppen werden mit Trifluoressigsäure entfernt und der synthetisierte PNA Strang wird mit Licht der Wellenlänge 365 nm abgespalten.

Die Synthese von PNA auf photoabspaltbaren Linkern kann auch großflächig auf dem gesamten Chip durchgeführt werden, wobei die Komponenten über den Chip gespült werden. Die Entschützung erfolgt wie beschrieben.

### Beispiel 5:

### Synthese von DNA auf Chips, die einen nichtphotoabspaltbaren Linker tragen

Im ersten Schritt werden die Glassubstrate chemisch modifiziert, so dass eine an sich bekannte gezielte Anbindung von Oligonukleotidbausteinen erfolgen kann. Dazu werden die Substrate silanisiert, wobei das Silan eine funktionalisierte (beispielsweise OH- oder NH₂-) Alkylkette trägt. Die Synthese des nichtphotoabspaltbaren Linkers erfolgt durch Kopplung von 5'-geschützten T, A, C oder G Monomeren mit Succinanhydrid und Dimethylaminopyridin. Als Schutzgruppen auf den Monomeren werden beispielsweise herkömmliche DMT- Gruppen für eine großflächige DNA Synthese oder photolabile Gruppen in Fall eines DNA-Arrays verwendet. Das zuvor behandelte Monomer wird mit dem Substrat gekoppelt und die Synthese, beispielsweise der Sequenz 5'-AGC CAG CTC ACT ACC TAG-3', wird wie in Beispiel 1 beschrieben durchgeführt. Die Abspaltung der 5'-Schutzgruppen wird entweder mit Trichloressigsäure oder durch Einstrahlung von Licht (365 nm) vorgenommen. Durch Einwirkung von Ammoniak Dampf wird die DNA vom Substrat abgelöst und der Array durch Blotting auf eine Nitrocellulosemembran übertragen.

### Beispiel 6:

### Blotting von DNA-Oligomer-Arrays

Um die aus Beispiel 5 von der modifizierten Glasoberfläche abgespaltenen Oligomerarrays an die Proben-DNA hybridisieren zu können, werden sie auf Nitrocellulose (Amersham Pharmacia Biotech, Hybond Serie, Nitrocellulose auf Nylon oder PVDF) geblottet, analog zur etablierten Methode des "Southern Blots". Dabei wird die Anordnung der Oligomere im Array nicht wesentlich verändert, sofern der Abstand der Punkte im Array ausreichend ist. Dazu wird der Glasträger mit der beladenen Seite auf einen Nitrocelluloseträger gelegt, welcher vorher mit einem Hybridisierungspuffer (SSC + SDS, Denhardt's Reagenz) angefeuchtet wird. Durch Ausüben von Druck kommt es zur Übertragung der Oligomere auf die Nitrocellulose. Auf der Nitrocellulose ist bereits die Proben-DNA immobilisiert, mit welcher dann die Oligomere spezifisch hybridisieren. Nicht nicht hybridisierten Oligomere werden ausgewaschen.

Die Detektion erfolgt über einen an den Oligomeren angebrachten Fluoreszenzmarker (siehe Beispiel 9).

### Beispiel 7:

### Synthese von PNA auf Chips, die einen nichtphotoabspaltbaren Linker tragen

Die Substratoberfläche wird silanisiert, wobei das Silan eine funktionalisierte (beispielsweise NH₂- oder Glycidoalkyl-) Alkylkette trägt. Anschließend erfolgt die Kopplung des Amins mit einer Carbonsäure eines nichtphotoabspaltbaren Linkers, beispielsweise eine geschützte Hydroxyalkylcarbonsäure oder des Epoxides unter sauren Bedingungen mit Ethylenglycolen unterschiedlicher Kettenlänge. Die PNA Synthese beispielsweise der Sequenz 5'-AGC CAG CTC ACT ACC TAG-3' wird wie im Beispiel 2 beschrieben durchgeführt. Die Kopplung des Monomers G mit dem endständigen Alkohol führt zur Ausbildung eines Esters. Die PNA Synthese kann auch mit einer photoabspaltbaren Schutzgruppe auf den Amino-Gruppen durchgeführt werden. Dabei erfolgt die Abspaltung anstatt mit 20 % Piperidin in DMF mit Licht der Wellenlänge 365 nm. Durch Einwirkung von Ammoniak Dampf wird die PNA vom Substrat abgelöst und wird durch Blotting auf Nitrocellulose mit der Proben-DNA kontaktiert.

### Beispiel 8:

### Blotting von PNA-Oligomer-Arrays

Das Verfahren zur Identifizierung von PNA Oligomeren ist ähnlich dem zur DNA-Identifizierung, es wird ein PNA-Hybridisierungspuffer verwendet. Dieses Verfahren ist analog zur etablierten Methode des "Western Blots" für Proteine.

### Beispiel 9:

### Koppeln eines Fluoreszenz-Farbstoffs auf PNA Oligomere

Um einen fluoreszierenden Farbstoff, bevorzugt sind Cy3 oder Cy5, an PNA Oligomere zu koppeln, wird der N-Terminus (die 5'-Aminogruppe) mit dem N-Hydroxysuccinimid-Derivat des Farbstoffes unter Bildung eines Amids umgesetzt. Dazu wird das Farbstoffderivat in DMF oder DMSO gelöst und in Gegenwart von Triethylammoniumhydrogencarbonat-Puffer mit den immobilisierten Oligomeren kontaktiert. Nach-2 h ist die Reaktion beendet und der überschüssige bzw. hydrolysierte Farbstoff wird durch Waschschritte mit Wasser und Methanol entfernt.

### Beispiel 10:

### Immobilisierung von PCR-Produkten auf modifizierten Glasoberflächen

Um PCR-Produkte auf Glasträgern immobilisieren zu können, werden sowohl die PCR-Produkte als auch Glasoberfläche modifiziert. An das 3'-Ende der PCR-Produkte wird ein A-minopropyl-Derivat gekoppelt, das eine freie Aminogruppe liefert. Die Glasoberfläche wird mit 3'-Glycidoxyalkyltrimethoxysilan und katalytischen Mengen Diisopropylethylamin derivatisiert und trägt ein Epoxid auf der Oberfläche. Die Immobilisierung erfolgt durch Spotten einer Lösung der PCR-Produkte in 0,1 M Kaliumhydroxid und Inkubation für 6 Stunden bei 37 °C und 100% Luftfeuchte. Anschließend werden nicht gebundene PCR-Produkte durch Waschen mit Wasser entfernt.

### Beispiel 11:

### Immobilisierung der Proben-DNA auf Nitrocellulose oder PVDF-Membranen

Die Immobilisierung der Proben-DNA auf Nitrocellulose oder PVDF-Membranen erfolgt nach den Angaben der Hersteller. Besonders geeignet sind Membranen, die bereits auf eine Glasoberfläche im Mikroskopträgerformat (z. B. von Schleicher und Schüll) aufgebracht wurden und sich daher nach der Hybridisierung in herkömmlichen Fluoreszenzscannern einfach analysieren lassen. Nach der Immobilisierung werden die Membranen mit Hybridisierungspuffer angefeuchtet.

## Patentansprüche

1. Verfahren zur Charakterisierung eines Nukleinsäurefragmentes, wobei man die folgenden Verfahrensschritte ausführt:
a) man immobilisiert das zu charakterisierende Nukleinsäurefragment auf einer Oberfläche;
b) man stellt auf einer zweiten Oberfläche einen Array von Oligomeren her, wobei die Oligomere mit einem Marker versehen sind;
c) man löst die synthetisierten Oligomere von der Oberfläche ab, ohne dass diese dabei einen vorgegebenen Bereich auf der Oberfläche zu verlassen;
d) man kontaktiert die Oberfläche, auf der die zu charakterisierende Nukleinsäure immobilisiert ist, mit der Oligomer Array Oberfläche wobei komplementäre Oligomere des Arrays an die zu charakterisierende DNA hybridisieren;
e) man entfernt nicht komplementäre Oligomere;
f) man detektiert die komplementären Oligomere mittels deren Marker, wobei man anhand des Ortes auf der Oberfläche Sequenzinformation ermittelt.

2. Verfahren gemäß Anspruch 1, **dadurch gekennzeichnet, dass** das zu charakterisierende Nukleinsäurefragment ein Amplifikat genomischer DNA ist.

3. Verfahren gemäß Anspruch 2, **dadurch gekennzeichnet, dass** man die genomische DNA vor der Amplifikation mit einer Lösung eins Bisulfits, Disulfits oder Hydrogensulfits umsetzt.

4. Verfahren gemäß einem der vorangehenden Ansprüche, **dadurch gekennzeichnet, dass** man das zu charakterisierende Nukleinsäurefragment kovalent an die Oberfläche bindet.

5. Verfahren gemäß Anspruch 4, **dadurch gekennzeichnet, dass** man in das charakterisierende Nukleinsäurefragment eine Aminofunktion einführt und diese an eine durch Silanisierung derivatisierte Glasoberfläche bindet.

6. Verfahren gemäß einem der voranstehenden Ansprüche, **dadurch gekennzeichnet, dass** man die Oligomere des Arrays kovalent an die zweite Oberfläche bindet.

7. Verfahren gemäß Anspruch 6, **dadurch gekennzeichnet, dass** man in die Oligomere des Arrays eine Aminofunktion einführt und diese an eine durch Silanisierung derivatisierte Glasoberfläche bindet.

8. Verfahren gemäß Anspruch 6 oder 7, **dadurch gekennzeichnet, dass** man den Oligomer Array durch Festphasensynthese der Oligomere auf der zweiten Oberfläche herstellt.

9. Verfahren gemäß Anspruch 8, **dadurch gekennzeichnet, dass** man die Festphasensynthese der Oligonukleotide auf der zweiten Oberfläche in einer geschlossenen Synthesekammer durchführt, in welche man selektiv Synthesereagenzien einspeist.

10. Verfahren gemäß Anspruch 9, **dadurch gekennzeichnet, dass** man die Synthese der Oligomere durch selektive Zuführung der Synthesereagenzien an die jeweiligen Orte, an denen die Oligomere synthetisiert werden, durchführt.

11. Verfahren gemäß einem der Ansprüche 6-10 **dadurch gekennzeichnet, dass** man photolithographische Methoden und photolabile Schutzgruppen zur Oligomersynthese verwendet.

12. Verfahren gemäß Anspruch 11, **dadurch gekennzeichnet, dass** man für das photolithographische Verfahren elektronisch ansteuerbare und/oder veränderbare Masken verwendet.

13. Verfahren gemäß Anspruch 11, **dadurch gekennzeichnet, dass** man für die photolithographische Verfahren ein selektiv schaltbaren Spiegelarray zur Erzeugung eines Belichtungsmusters verwendet.

14. Verfahren gemäß einem der Ansprüche 6-13, **dadurch gekennzeichnet, dass** man die Synthese der Oligomere in einem Array von Kavitäten durchführt, welche man gegebenenfalls weiterhin als Kammern für die Hybridisierung verwendet.

15. Verfahren gemäß einem der Ansprüche 1 bis 13, **dadurch gekennzeichnet, dass** man die Immobilisierung der zu charakterisierenden Nukleinsäurefragmente an einen Array von Kavitäten durchführt, welche man gegebenenfalls weiterhin als Kammern für die Hybridisierung verwendet.

16. Verfahren gemäß einem der voranstehenden Ansprüche, **dadurch gekennzeichnet, dass** man als Marker der Oligomere chemische Gruppen verwendet, welche eine Massenveränderung und/oder Fluoreszenz bewirken.

17. Verfahren gemäß einem der vorangehenden Ansprüche **dadurch gekennzeichnet, dass** man die Detektion der hybridisierten Oligomere mittels Massenspektrometrie, vorzugsweise mittels Matrix assistierter Laser Desorptions/Ionisations Massenspektrometrie (MALDI), durchführt.

18. Verfahren nach einem der vorangehenden Ansprüche, **dadurch gekennzeichnet, dass** man über die Identität der hybridisierten Oligomere Information über Cytosin Methylierungen in einer genomischen DNA Probe ermittelt.

## Claims

1. A method for characterizing a nucleic acid fragment, wherein the following method steps are conducted:
a) the nucleic acid fragment to be characterized is immobilized on a surface;
b) an array of oligomers is prepared on a second surface, whereby the oligomers are provided with a label;
c) the synthesized oligomers are stripped from the surface without leaving a pregiven region on the surface;
d) the surface on which the nucleic acid to be characterized is immobilized is contacted with the surface of the oligomer array, whereby complementary oligomers of the array hybridize to the DNA to be characterized;
e) non-complementary oligomers are removed;
f) the complementary oligomers are detected by means of their label, whereby sequence information is determined on the basis of the site on the surface.

2. The method according to claim 1, further **characterized in that** the nucleic acid fragment to be characterized is an amplified product of genomic DNA.

3. The method according to claim 2, further **characterized in that** the genomic DNA is reacted with a solution of bisulfite, disulfite or hydrogen sulfite prior to the amplification.

4. The method according to one of the preceding claims, further **characterized in that** the nucleic acid fragment to be characterized is bound covalently to the surface.

5. The method according to claim 4, further **characterized in that** an amino function is introduced into the nucleic acid fragment to be characterized and this function binds to a glass surface derivatized by silanizing.

6. The method according to one of the preceding claims, further **characterized in that** the oligomers of the array bind covalently to the second surface.

7. The method according to claim 6, further **characterized in that** an amino function is introduced into the oligomers of the array and this binds to a glass surface derivatized by silanizing.

8. The method according to claim 6 or 7, further **characterized in that** the oligomer array is produced by solid-phase synthesis of the oligomers on the second surface.

9. The method according to claim 8, further **characterized in that** the solid-phase synthesis of the oligonucleotides on the second surface is conducted in a closed synthesis chamber, and synthesis reagents are selectively introduced into this chamber.

10. The method according to claim 9, further **characterized in that** the oligomers are synthesized by the selective introduction of synthesis reagents to the respective sites at which the oligomers are synthesized.

11. The method according to one of the preceding claims, further **characterized in that** photolithographic methods and photolabile protective groups are used for the oligomer synthesis.

12. The method according to claim 11, further **characterized in that** electronically controllable and/or changeable masks are used for the photolithographic method.

13. The method according to claim 11, further **characterized in that** a mirror array, which can be switched on selectively for producing an exposure pattern, is used for the photolithographic method.

14. The method according to one of the claims 6 to 13, further **characterized in that** the oligomers are synthesized in an array of cavities, which are also used, if needed, as chambers for the hybridization.

15. The method according to one of claims 1 to 13, further **characterized in that** the nucleic acid fragments to be characterized are immobilized on an array of cavities, which are also used, if needed, as chambers for the hybridization.

16. The method according to one of the preceding claims, further **characterized in that** chemical groups, which effect a change in mass and/or fluorescence, are used as labels for the oligomers.

17. The method according to one of the preceding claims, further **characterized in that** the hybridized oligomers are detected by means of mass spectrometry, preferably by means of matrix-assisted laser desorption/ionization mass spectrometry (MALDI) .

18. The method according to one of the preceding claims, further **characterized in that** information on cytosine methylations in a genomic DNA sample is determined.

## Revendications

1. Procédé de caractérisation d'un fragment d'acide nucléique, dans lequel on réalise les étapes de procédé suivantes :
a) on immobilise le fragment d'acide nucléique à caractériser sur une surface ;
b) on prépare sur une deuxième surface un ensemble d'oligomères, dans lequel les oligomères sont pourvus d'un marqueur ;
c) on détache les oligomères synthétiques de la surface, mais sans que ceux-ci quittent une zone prédéterminée sur la surface ;
d) on met la surface sur laquelle l'acide nucléique à caractériser est immobilisé en contact avec la surface de l'ensemble d'oligomères, les oligomères complémentaires de l'ensemble s'hybridant aux ADN à caractériser ;
e) on supprime les oligomères non complémentaires ;
f) on détecte les oligomères complémentaires au moyen de leurs marqueurs, en déterminant des informations sur la séquence à partir de leur emplacement sur la surface.

2. Procédé selon la revendication 1, **caractérisé en ce que** le fragment d'acide nucléique à caractériser est un amplificat d'ADN génomique.

3. Procédé selon la revendication 2, **caractérisé en ce qu'**on convertit l'ADN génomique avant l'amplification par une solution de bisulfite, de disulfite ou d'hydrogénosulfite.

4. Procédé selon l'une quelconque des revendications précédentes, **caractérisé en ce qu'**on lie le fragment d'acide nucléique à la surface par covalence.

5. Procédé selon la revendication 4, **caractérisé en ce qu'**on introduit dans le fragment d'acide nucléique à caractériser une fonction aminée, et qu'on lie celle-ci à une surface en verre dérivée par silanisation.

6. Procédé selon l'une quelconque des revendications précédentes, **caractérisé en ce qu'**on lie les oligomères de l'ensemble à la deuxième surface par covalence.

7. Procédé selon la revendication 6, **caractérisé en ce qu'**on introduit dans les oligomères de l'ensemble une fonction aminée, et qu'on lie celle-ci à une surface de verre dérivée par silanisation.

8. Procédé selon la revendication 6 ou 7, **caractérisé en ce qu'**on prépare l'ensemble d'oligomères par synthèse en phase solide des oligomères sur la deuxième surface.

9. Procédé selon la revendication 8, **caractérisé en ce qu'**on effectue la synthèse en phase solide des oligonucléotides sur la deuxième surface dans une chambre de synthèse fermée, qu'on alimente sélectivement en réactifs de synthèse.

10. Procédé selon la revendication 9, **caractérisé en ce qu'**on effectue la synthèse des oligomères par apport sélectif des réactifs de synthèse aux endroits respectifs auxquels les oligomères sont synthétisés.

11. Procédé selon l'une quelconque des revendications 6 à 10, **caractérisé en ce qu'**on utilise des procédés photolithographiques et des groupes de protection photolabiles pour la synthèse des oligomères.

12. Procédé selon la revendication 11, **caractérisé en ce qu'**on utilise pour le procédé photolithographique des masques réglables et/ou modifiables électroniquement.

13. Procédé selon la revendication 11, **caractérisé en ce qu'**on utilise pour le procédé photolithographique un ensemble de miroirs sélectivement commutables pour produire un type d'éclairage.

14. Procédé selon l'une quelconque des revendications 6 à 13, **caractérisé en ce qu'**on effectue la synthèse des oligomères dans un ensemble de cavités, qu'on utilise éventuellement aussi comme chambres pour l'hybridation.

15. Procédé selon l'une quelconque des revendications 1 à 13, **caractérisé en ce qu'**on effectue l'immobilisation des fragments d'acide nucléique à caractériser dans un ensemble de cavités, qu'on utilise éventuellement aussi comme chambres pour l'hybridation.

16. Procédé selon l'une quelconque des revendications précédentes, **caractérisé en ce qu'**on utilise comme marqueurs des oligomères des groupes chimiques qui provoquent un changement des masses et/ou de la fluorescence.

17. Procédé selon l'une quelconque des revendications précédentes, **caractérisé en ce qu'**on effectue la détection des oligomères hybridés au moyen de la spectrométrie de masse, de préférence au moyen de la spectrométrie de masse à désorption-ionisation par lasers assistés par matrice (MALDI).

18. Procédé selon l'une quelconque des revendications précédentes, **caractérisé en ce que**, grâce à l'identité des oligomères hybridés, on obtient des informations sur les méthylisations de cytosine dans un échantillon d'ADN génomique.
